# EUROPEAN PATENT SPECIFICATION

(11) **EP 2 956 085 B1**
(45) Date of publication and mention of the grant of the patent: **01.04.2020**
(21) Application number: 14708673.0
(22) Date of filing: 13.02.2014
(51) Int. Cl.: A61F 2/00

(54) **DEVICE FOR ANAL CERCLAGE**
VORRICHTUNG FÜR EINEN ANAL-UMSCHLINGUNGSDRAHT
DISPOSITIF DE CERCLAGE ANAL

(30) Priority: 14.02.2013 IT MO20130034
(43) Date of publication of application: 23.12.2015
(73) Proprietor: THD S.p.A., 42015 Correggio, Reggio Emilia (IT)
(72) Inventor: BASTIA, Filippo, I-41019 Soliera (modena) (IT); DEVESA MUGICA, José Manuel, E-28109 Madrid (ES)
(74) Representative: Paparo, Aldo
(86) International application number: PCT/IB2014/058954
(87) International publication number: WO 2014/125425

(56) References cited:
- WO-A1-01/52777
- WO-A1-2004/066887
- WO-A1-2005/082275
- US-A1- 2005 277 963
- US-A1- 2007 173 685
- US-A1- 2011 098 731

## Description

The present invention relates to a device for anal cerclage.

Anal cerclage means a radial reduction of the cross section of the anal canal.

It is here described also a surgical method for the recovery of sphincter tone.

The terminal zone of the rectum has above it a rectal ampulla, inside which gas and faeces are accumulated, and which continues with the rectal canal terminating on the outside with the anus.

The rectal canal is peripherally encircled by a first muscular wall, called internal sphincter, and a second muscular wall, called external sphincter.

In other words, the anal canal is encircled by a first ring-shaped muscle layer, the internal sphincter, and by a second ring-shaped muscle layer, the external sphincter, the latter surrounding the first.

In patients with regular muscular wall tone, the internal sphincter, involuntary, is normally closed, whereas the external sphincter, being voluntary, can be contracted or expanded by the patient.

In the rectal ampulla, there are receptors which detect the presence/absence of gas or faeces inside it, sending signals to the brain.

When the rectal ampulla is partially filled, the receptors detect the presence of gas or faeces inside it and send a signal to the brain.

As soon as the patient is in a condition to free the rectal ampulla, the defecatory act begins.

The defecatory act is a combination of reflex and involuntary actions that involve the two sphincters.

Some people are affected by a pathology called faecal incontinence, as a result of which they are unable to contract and/or maintain adequate sphincter tone and are therefore unable to retain the contents of the rectal ampulla inside the latter.

According to the severity of the pathology - severity which can depend, for example, on normal deterioration of the muscular walls or damage caused during a surgical intervention - there are conditions in which patients may have a malfunctioning internal sphincter muscle and thus find themselves in a condition of not being able to maintain the anal canal closed normally.

There are degrees of faecal incontinence in which the reflexive sphincter tone, controlled by the patient, is compromised, and degrees of faecal incontinence, much more severe than the previous ones, in which the basal sphincter tone, i.e. the tone of the internal sphincter muscle, is also compromised.

Documents US2007/173685, US2011/098731 or US2005/277963, describe cerclage devices having elements designed to lock the device in a round configuration, wherein the locking elements are configured to provide the locking according to a fixed configuration, namely a predetermined outline or diameter of the device in an operative configuration.

Moreover, it is known from WO01/52777 a cerclage device having one end provided with an inflatable locking element which can be inserted in an opposite hollow end of the device, wherein the inflatable locking element can be inflated and therefore locked in the hollow end to determine a desired configuration of the device in operation.

In this context, the technical task at the basis of the present invention is to propose a device for the cerclage of the anal canal and for the recovery of sphincter tone.

In particular, it is an object of the present invention to provide a device capable of generating an elastic containment of the anal canal in order to reconstruct sphincter tone.

The stated technical task and specified objects are substantially achieved by a device capable of generating a cerclage of the anal canal to recover the sphincter tone, comprising the technical features set forth in one or more of the appended claims.

Additional features and advantages of the present invention will appear more clearly from the approximate, and thus not limiting, description of a preferred but not exclusive embodiment of a device for the cerclage of the anal canal and of an associated method for the recovery of sphincter tone, as illustrated in the appended drawings, in which:

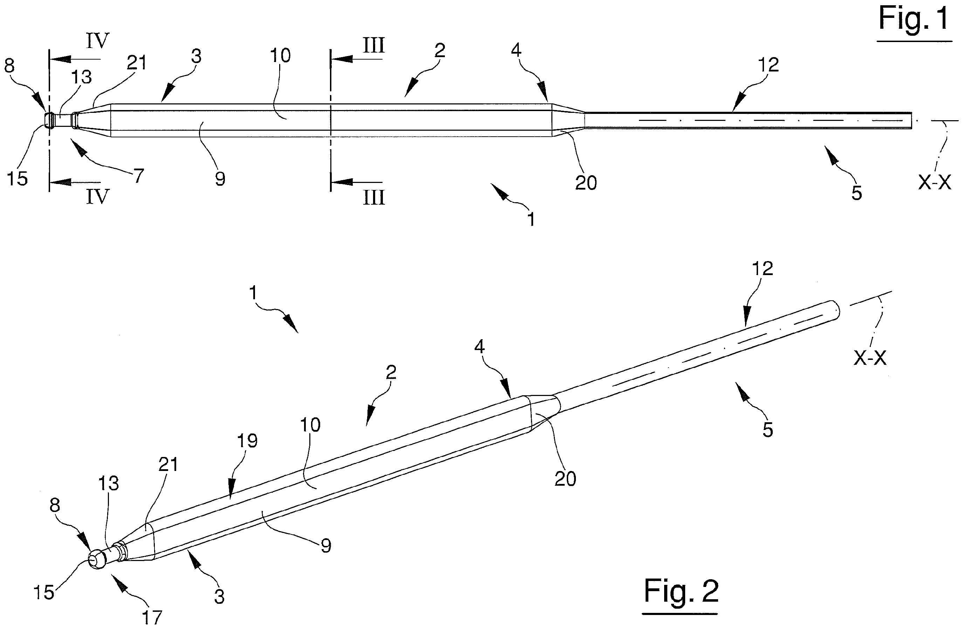

- figure 1 is a side view of a device for the cerclage of the anal canal according to an embodiment of present invention, in the rest configuration;
- figure 2 is a perspective view of the device in figure 1;
- figure 3 is a cross-sectional view of a constructive detail of the device for the cerclage of the anal canal, drawn on a plane III-III of figure 1;
- figure 4 is a cross-sectional view of another constructive detail of the device for the cerclage of the anal canal, drawn on a plane IV-IV of figure 1;
- figure 5 is a plan view of the device for the cerclage of the anal canal in a first active configuration in a contact position;
- figure 6 is a plan view of the device for the cerclage of the anal canal in the active configuration of figure 5, but in a constraint position;
- figure 7 is a first operational step of a method for the recovery of sphincter tone using a device according to the present invention;
- figure 8 is a second operational step of the method;
- figure 9 is a third operational step of the method;
- figure 10 is a fourth operational step of the method;
- figure 11 is a fifth operational step of the method;
- figure 12 is a sixth operational step of the method;
- figure 13 is a seventh operational step of the method;
- figure 14 is a cross section of an anal canal illustrating, in the left section, the condition prior to insertion of the device, and, in the right section, the condition following insertion of the device.

With reference to the aforesaid figures, 1 indicates overall a device for the cerclage of the anal canal CA.

Anal canal CA means the terminal tract of the rectum R, situated below the rectal ampulla AR, extending along an axis Y-Y and having an initial cross section B.

Said anal canal CA, terminates outside the rectum R with the anus A, encircled by external tissue TE.

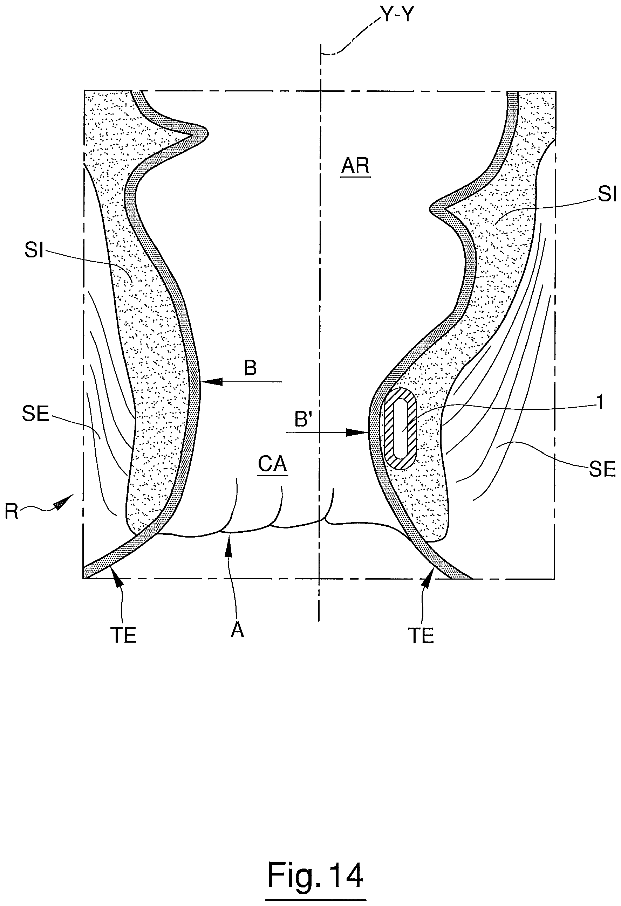

Said anal canal CA, illustrated in figure 14, has a substantially cylindrical shape and is encircled by a first internal muscular wall SI, also called internal sphincter, and a second external muscular wall SE, also called external sphincter.

In other words the anal canal CA can be idealised as a hollow ring-shaped body extending along the axis Y-Y, covered around the whole of its outer periphery by a first closed circular wall, defined by the internal sphincter SI, which is in turn covered around its outer periphery by a second closed circular wall, defined external sphincter SE.

Cerclage of the anal canal CA means narrowing the cross section B along a tract thereof by applying the device 1.

In other words, the application of the device 1 generates a compression of the anal canal CA, which brings the cross section thereof, at least along one tract, from an initial section B to a final cross section B', the former being larger than the latter.

Said device 1 comprises a central tract 2, a first tract 7 and locking means 16, and can comprise a second tract 5.

Said device can take on a rest configuration and an active configuration when applied to the anal canal CA.

In the rest configuration, the central tract 2, second tract 5 and first tract 7 can extend along a longitudinal axis X-X.

In the active configuration, the central tract 2 and first tract 7 take on a closed ring shape subtending an area C and a closed ring-shaped contour K-K.

Said closed ring-shaped contour K-K can take on an ellipsoidal shape, or more generically an irregular arched shape defining in any case a closed contour.

The device 1 is preferably made of yielding elastic material, such as, for example, silicone, or another plastic material.

In the rest configuration, the central tract 2 extends along a longitudinal axis X-X and terminates at either end with a first terminal end 3 and with a second terminal end 4. The first and second terminal ends are opposite each other.

The central tract 2 has within it a cavity 6, preferably rectangular in shape.

Said cavity 6 preferably extends along the whole length of the central tract 2 along a longitudinal axis X-X.

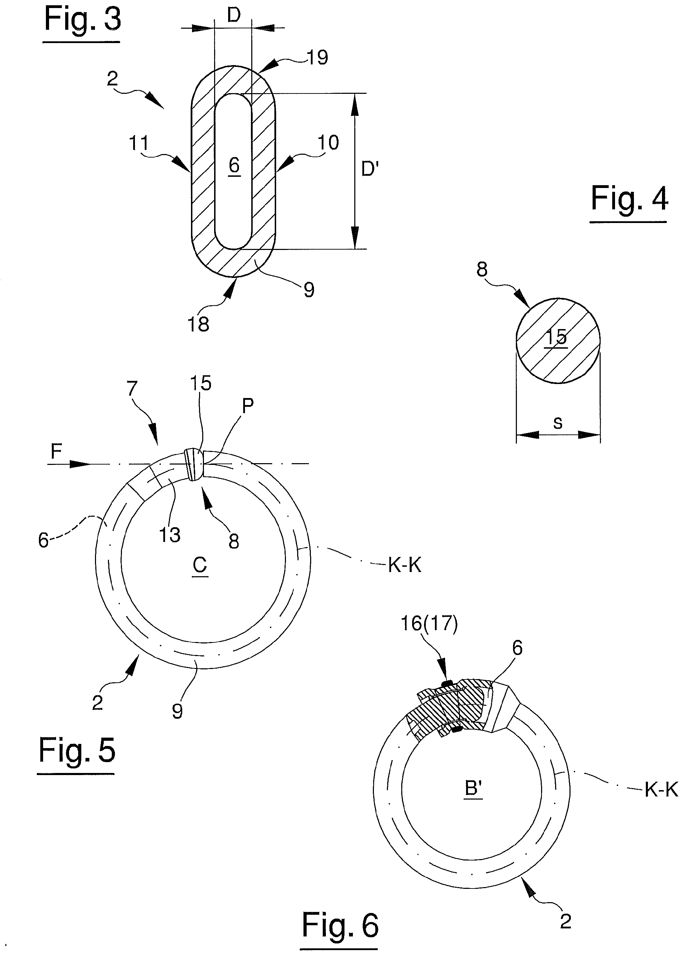

Said cavity 6 has a distance D, transversely relative to the longitudinal axis X-X, and a distance D', longitudinally relative to the longitudinal axis X-X (illustrated in figure 3).

Said central tract 2 is defined by a strip 9. Said strip 9 has an internal wall 10 and an external wall 11. The internal wall 10 and external wall 11 are opposite each other.

The internal wall and external wall 10, 11 are connected by a pair of connecting walls 18, 19 opposite each other.

The internal wall and external wall 10, 11 subtend within them a distance D, while the pair of connecting walls 18, 19 subtend within them a distance D'.

As illustrated in figure 3, the cross section of the strip 9, defined by the intersection with a plane III-III which is perpendicular to the longitudinal axis X-X, has a substantially rectangular outer contour. By virtue of the preferred hollow shape of the strip 9, the cross section of the strip 9 defines a framing contour formed by the walls 10, 11, 18 and 19.

The rectangular cross section of the strip 9 is preferable for forming a band for cerclage of the anal cavity CA.

In other words, when the strip is placed around the axis Y-Y of the anal canal CA, the internal wall 10 will encircle the latter uniformly.

In fact, in the active configuration of the device 1, better described below, the internal wall 10 is always facing the inside of the closed ring-shaped contour K-K.

At the first terminal end 3 there is the first tract 7. Said first tract 7 is integrally connected to the central tract 2 at the first terminal end 3, by means of a first connecting portion 21.

Said first tract 7, which extends the first terminal end 3, has an engagement element 8.

As can be seen in figure 4, the engagement element 8 subtends a distance S at least in a cross section thereof.

In other words, the engagement element 8, as it extends along a longitudinal axis X-X, if intersected by a perpendicular plane IV-IV, will subtend a distance S at least along said plane.

The first tract 7 is preferably defined by a cylindrical body 13, having a direction of extension along a longitudinal axis X-X.

The cylindrical body 13 ends with the engagement element 8.

Said engagement element 8 is preferably defined by a spherical body 15 with a diametrical section equal to S.

Said diametrical section S, i.e. said distance S subtended by the engagement element 8, is larger than at least one of the distances D and D' of the cavity 6.

In a preferred embodiment at the second terminal end 4 of the central tract of the device in the rest configuration there is the second tract 5.

Said second tract 5 is integrally connected to the central tract 2, at the second terminal end 4, by means of a second connecting portion 20.

Said second tract 5, extending the second terminal end 4, is preferably defined by a hollow tubular body 12 having a direction of extension along a longitudinal axis X-X.

Said second tract 5 acts as an element for drawing the device 1 when it is inserted inside the anal cavity CA.

Coming back to the device 1, the rest configuration is taken on when the first tract 7 and the second terminal end of the central tract 2 are uncoupled from each other.

Conversely, the active configuration is taken on when the first tract 7 and the second terminal end of the central tract 2 are coupled to each other.

In this configuration, the first tract 7 and central tract 2 lend the device a closed ring shape, subtending within it an area C that is smaller than the cross-sectional area B of the anal canal CA.

In this active configuration, the two tracts 7,2 create an interpenetration coupling, defined by the insertion of the engagement element 8 within the cavity 6.

In this active configuration, the engagement element 8 is entirely inserted within the cavity 6.

Said engagement element 8 is thus contained within the cavity 6.

Considering the active configuration in detail, the latter is divided into a first transient phase, which results in a contact position illustrated in figure 5, and a second transient phase, which results in a constraint position illustrated in figure 6.

In the contact position, the engagement element 8 comes into contact in the cavity 6 in at least one point P defining the closed ring-shaped contour K-K.

In the constraint position, the engagement element 8 translates within the cavity 6, becoming inserted inside it.

The translation of the engagement element 8 takes place along a direction F tangent to the point P of contact.

With particular reference to the preferred embodiment, the contact position of the active configuration of the device occurs through the positioning of the spherical body 15 in front of the cavity 6, which places them in contact, whereas the constraint position, also in the active configuration, and following the preceding one, occurs with the insertion of the spherical body 15 within the cavity 6, until it is contained entirely inside the cavity 6.

By exploiting the length of the cavity 6, it is possible to achieve a translation in the degree to which the engagement element 8 is received that varies according to the anatomical configuration of the patient's anal canal CA.

In fact, based on the anatomical configuration of the patient's anal canal A and the detected degree of severity of the incontinence, it will be possible to vary the translation of the engagement element 8 inside the cavity 6 as desired.

In this manner, by adjusting the depth of penetration of the engagement element 8 inside the cavity 6, it will be possible to dimension the closed ring-shaped contour K-K of the device 1 as desired, according to how much one wishes to compress the initial cross section B of the anal canal CA.

Subsequently, it will be possible to apply locking means 16 between the first tract 7 and the central tract 2 so as to prevent them from sliding.

Said locking means 16 are active on the central tract 2, outside the strip 9, to determine a squeezing of the portion of the cavity 6. This squeezing prevents the spherical body 15 from coming out of the cavity 6.

In other words, the squeezing of the locking means 16, localized at the strip 9, compresses the central tract 2, reducing the respective distances D, D' subtended between the walls of the strip.

In this manner, the locking means 16, besides creating an integral coupling between the central tract and first tract 2, 7 reduces, upstream of the spherical body 15, the distances D, D' subtended by the walls of the strip 9, preventing the passage of the spherical body 15 within the cavity 6.

Said locking means 16 are preferably defined by at least one band 17, adjustable in the gripping section.

In the preferred embodiment illustrated in the appended figures, the locking means 16 are defined by a plurality of bands 17.

With reference to the method for the recovery of sphincter tone, the latter is implemented by means of the device 1, a surgical instrument 23, fixing means 24 and a sensor 25 for sensing the nominal radial pressure (PRN) and the desired radial pressure (PRD) of the anal canal CA.

The surgical instrument 23 is commonly called a cap-hook in the art, and has a tip 26 that can be inserted in the external tissue TE of the anal cavity CA and simultaneously engaged by interference with the device 1 as the tip 26 is inserted within the cavity 6 of the second tract 5.

The fixing means 24 are preferably defined by a clip 27 preferably having a first engagement seat 28 and a second engagement seat 28'.

The first engagement seat 28 is engageable, outside the anal cavity CA, with the first tract 7, whereas the second engagement seat 28' is engageable, likewise outside the anal cavity CA, with the second tract 5 or the central tract 2.

The sensor 25 can also simply be defined by the surgeon's finger which, inserted in the anal cavity CA, senses the nominal radial pressure exerted by the anal cavity CA on the surgeon's finger.

Said sensor 25 can also consist in a probe 29, which can be inserted in the anal cavity CA so as to automatically measure, in a known manner, the nominal radial pressure of the anal cavity A.

The method of intervention for the recovery of sphincter tone comprises the following steps:
- creating at least a first and a second incision G, G' in the external tissue TE of the anus A;
- inserting the surgical instrument 23 between the first and the second incision G,G', creating a first connection channel H between them and making the tip 26 exit from the second incision G';
- coupling the second tract 5 with the surgical instrument 23;
- extracting the surgical instrument 23 from the first connection channel H, favouring the insertion of the central tract 2 within the first connection channel H;
- repeating the insertion, coupling and extraction steps at least once, thereby creating at least a second connection channel H' between the second and first incision G,G'; said central tract 2 being partly inserted within the connection channels H,H', thus creating a closed ring-shaped contour K-K, and partly, along with said first tract and first tract 7,5, coming out from the incisions G;
- constraining the second tract 5 and the first tract 7 on the fixing means 24;
- pulling the second tract 7 until reaching a pre-defined contour K-K and an area C subtended by the device so as to give the anal cavity A a desired radial pressure value (PRD);
- cutting an excess portion of the central tract 2 and first tract 7 projecting from the incision G, thus freeing the cavity 6;
- coupling the engagement element 8 of the first tract 7 within the cavity 6 of the central tract 2;
- locking the first and second tracts 6, 7 to each other by applying locking means 16 on the first tract 7.

The above-described method can envisage, prior to the step of pulling the second tract, the following preliminary step:
- inserting the sensor 25 within the anal cavity CA to measure a nominal radial pressure PRN.

The method of intervention is described here below.

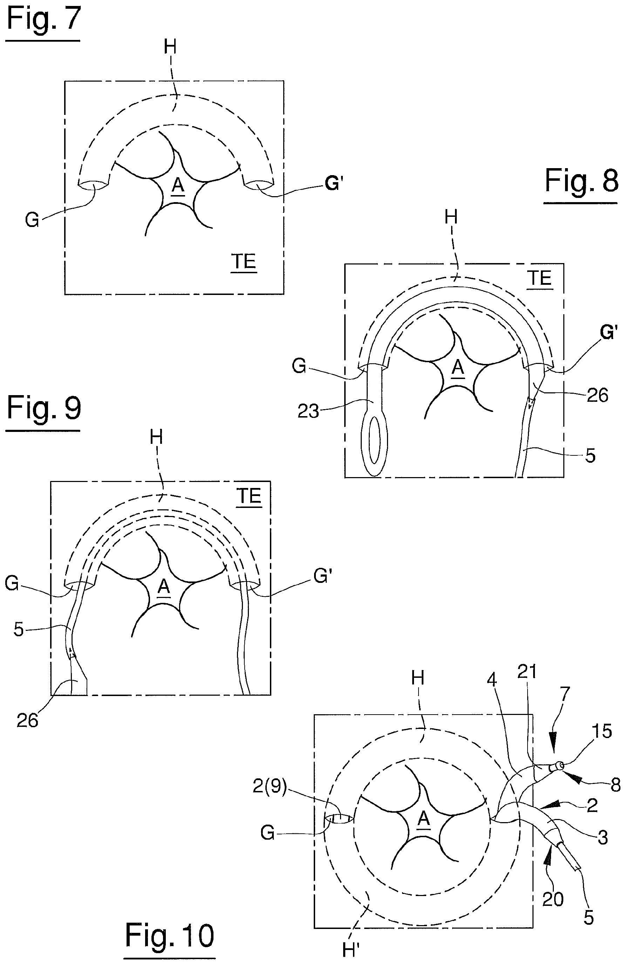

As illustrated in figure 7, the incision step is carried out by making at least two, preferably four, incisions G,G'.

Preferably, in order to achieve a better hold, the number of incisions made is four.

Said incisions are made in a circumferential perimeter about three centimetres away from the anus A.

Said incisions, preferably made in a number of four, are located, if we idealise the application of a clock dial on the external tissue TE, at 2, 5, 7 and 10 o'clock.

As illustrated in figure 8, once the incisions G, G' are made, the surgeon proceeds to insert of the surgical instrument 23 between the first and the second incision G, G', thereby creating a connection channel H between them, situated below the external wall TE of the anus A.

After making the tip 26 of the surgical instrument exit from the second incision G', one proceeds by coupling the second tract 5 to the same.

This coupling is achieved by inserting the tip 26 within the cavity 6.

Subsequently, the surgical instrument 23 is extracted, thus favouring the insertion of the second tract 5 within the first channel H.

Better positioning of the device 1 is achieved by pulling the second tract 5 until a portion of the central tract 2 is placed within the first channel H.

Subsequently, the surgical instrument is inserted between the second and the first incision G',G to create a second connection tract H'.

The second tract 5 is coupled once again to the surgical instrument 23 and the latter is again extracted, thus favouring the insertion of the first tract 7 within the second channel H'.

In this case as well, better positioning of the device 1 is achieved by pulling the second tract 5 until a portion of the central tract 2 is placed within the second channel H'.

On completion of these preliminary steps, one reaches a configuration of the patient's anus A as illustrated in figure 10, where on the periphery of the anus A there are: a plurality of channels H, H', the central tract 2 passing through them, and an incision G', from which the first and second tract 5,7 and a portion of the central tract 2 project.

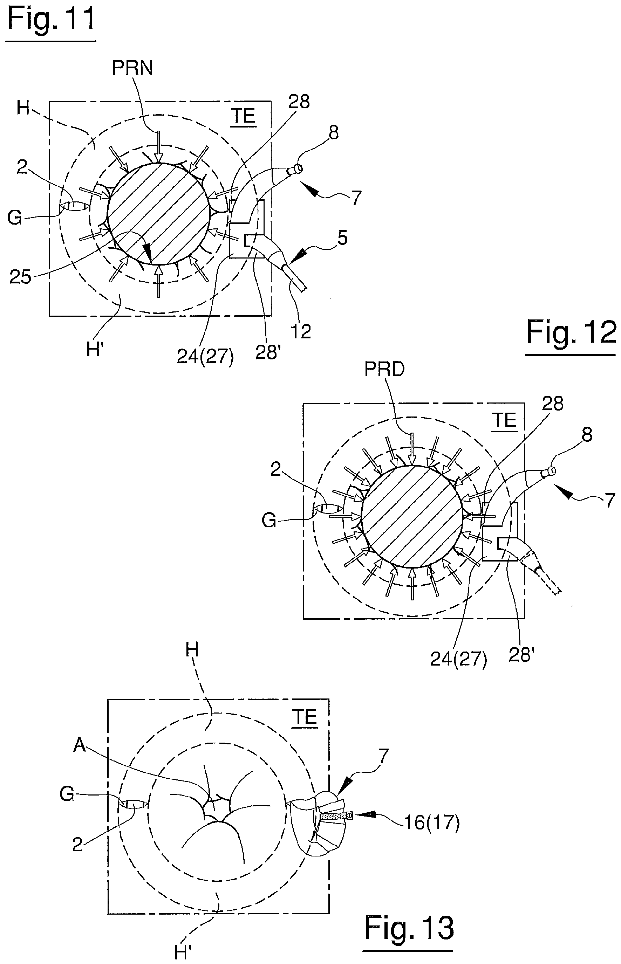

As illustrated in figure 11, one proceeds to fix the flaps of the first and of the second tract 7, 5, using appropriate fixing means 24.

In particular, the second tract 5 is coupled to the first engagement seat 28 and the first tract 7 is coupled to the second engagement seat 28'.

More precisely, during the fixing of the flaps of the first and second tracts 7, 5, the engagement element 8 is placed in abutment against the respective first engagement seat 28 and a portion of the second tract 5 in abutment against the respective second engagement seat 28'.

Subsequently, the radial pressure of the anal cavity CA is measured by inserting the sensor 25.

After the radial pressure of the anal cavity CA has been measured, the surgeon determines how much the device 1 inserted within the connection channels H, H' needs to be tightened.

In order to narrow the area C subtended by the device 1, the flap of the second tract 5 is pulled.

In this transient phase the resistance of the engagement element 8, or the spherical body 15, in abutment against the first engagement seat 28 of the fixing means 24, prevents the device 1 from being disengaged from the connection channels H, H'.

The second tract 5 is pulled until the pre-established radial pressure of the anal canal is reached.

In other words, in order to restore a predetermined muscle tone, the surgeon will evaluate how much the second tract 5 needs to be pulled.

When the pre-established muscle tone is restored, an excess portion of the central tract 2 is removed, along with, consequently, the second tract 5 connected to it. In other words, a portion of the strip 9 is cut so that the central tract and second tract will be opposing the first tract 7, and, more precisely, in front of the spherical body 15.

In particular, it is preferable to cut the central tract 2 leaving a twelve millimetre long portion of the second tract 5 from the point of constraint with the fixing means 24.

Subsequently, the first tract 7 is coupled to the central tract 2.

This coupling takes places in a first transient phase creating a first contact position and in a second transient phase creating a second constraint position.

In the contact position, the engagement element 8 is placed in contact, in at least one point P, with the cavity 6.

In this transient phase, the device 1 defines a closed ring-shaped contour K-K.

In the constraint position, the engagement element 8 is inserted within the cavity 6.

This step is achieved by translating the engagement element 8 completely within the cavity 6.

Said translation takes place along a direction F tangent to the previous point of contact P.

Once the coupling between the first tract and central tract 7,2 has been achieved, in this case between the spherical body 15 within the cavity 6, the locking means 16 are applied.

By applying the locking means, i.e. the plurality of bands 17, one achieves an indissoluble connection between the first tract and central tract 2,7.

The device for the cerclage of the anal canal and the method of intervention for the recovery of sphincter tone achieve important advantages.

The device has a very long useful life as, given the particular configuration of the system for coupling the anchorage element to the cavity, and is potentially less subject to shear forces which, due to the nature of the material, can compromise the correct functioning of the device when inserted into the patient's anal cavity.

Secondly, the interpenetration between the anchorage element and cavity makes it possible to adjust the contour K-K and the area C defined by the device as desired and hence in relation to the anatomy of the patient's anal canal and the degree of recovery that the patient requires.

## Claims

1. A device for the cerclage of an anal canal (CA), said anal canal (CA) having an extension along an axis (Y-Y) and an initial cross section (B) and being encircled around its periphery by a first internal muscular wall (SI) and a second external muscular wall (SE) encircling the previous one, comprising:
- a central tract (2) defined by at least two opposing walls (10, 11) and having within it a cavity (6) having a width (D) transversally, at least in one portion; said width (D) being defined, within the cavity (6), by the distance between the two opposing walls (10, 11), the central tract (2) terminating at either end with a first terminal end (3) and a second terminal end (4) opposite each other;
- a first tract (7), integrally connected to the central tract (2) at said first terminal end (3) by means of a connecting portion (21) and extending the central tract (2), the first tract (7) ending with an engagement element (8) having a transversal width, at least for a tract, of size (S); said size (S) preferably being larger than the width (D) of the cavity (6);
said device having a rest configuration, in which the central tract extends along a longitudinal axis (X-X) and the second terminal end (4) of the central tract and the first tract (7) are uncoupled from each other, and an active configuration, in which the central tract and first tract (2, 7) are coupled with each other, giving the device a closed ring shape subtending an area (C) within it defining a smaller final cross-sectional area (B') on the anal canal (CA) than the initial cross-sectional area (B);
**wherein** said central tract and said first tract (2, 7) create, in the active configuration of the device, an interpenetration coupling defined by the insertion of the engagement element (8) within the cavity (6),
wherein the device further comprises locking means (16) active between the first tract and the central tract (7, 2) to prevent sliding between them,
wherein said engagement element (8) is defined bv a spherical body (15) with a diametrical section equal to (S), and wherein said locking means (16) are active on the central tract (2) to define a squeezing of the cavity (6) upstream of the engagement element (8); said squeezing preventing the spherical body (15) from coming out from the cavity (6),
and in that said first tract (7) is defined by a cylindrical tract (13), having a direction of extension along a longitudinal axis (X-X), ending with the engagement element (8), and in that the cavity (6) extends along the whole length of the central tract (2) along a longitudinal axis (X-X) thereof so that the translation of the spherical body (8) inside the cavity (6) can be varied as desired and, bv adjusting the depth of penetration of the spherical body (8) inside the cavity (6), it is possible to dimension the closed ring-shaped contour (K-K) of the device (1) as desired, according to how much one wishes to compress the initial cross section (B) of the anal canal (CA).

2. The device according to claim 1, **characterised in that** in the active configuration said engagement element (8) is entirely contained within the cavity (6).

3. The device according to claim 1, **characterised in that** said active configuration envisages a preliminary step, wherein the first tract and central tract (7, 2) create a contact position wherein said engagement element (8) comes into contact in at least one point (P) with the cavity (6), defining a closed ring-shaped contour (K-K) and a second terminal step, wherein the first tract and central tract (7, 2) create a constraint position wherein said engagement element (8) translates within the cavity (6); said translation taking place along the direction (F) tangent to the point of contact (P).

4. The device according to claim 1, **characterised in that** said first tract and central tract (7, 2) are made of yielding elastic material, preferably silicone.

5. The device according to claim 1, **characterised in that** said central tract (2) is defined by a strip (9) having a direction of extension along a longitudinal axis (X-X), having a rectangular contour in its cross section, and defining a first internal wall (10) and a second external wall (11), said first and second wall (10,11) subtending a distance (D) within them inside the cavity (6).

6. The device according to claim 5, **characterised in that** said strip (9) has, when the device takes on the active configuration, its internal wall (10) facing the inside of the closed ring shaped contour (K-K).

7. The device according to claim 1, **characterised in that** said locking means (16) are defined by at least one band (17) active on the central tract (2).

8. The device according to claim 1, **characterised in that** said locking means (16), active on the central tract (2), promote a shortening of the distance (D).

9. The device according to claim 1, **characterised in that** said coupling between the engagement element (8) and cavity (6) is of the interference type.

## Patentansprüche

1. Vorrichtung für den Umschlingungsdraht eines Analkanals (CA), wobei der Analkanal (CA) eine Erstreckung entlang einer Achse (Y-Y) und einen Anfangsquerschnitt (B) aufweist und um seinen Umfang von einer ersten inneren Muskelwand (SI) und einer zweiten äußeren Muskelwand (SE), die die vorhergehende umschlingt, umschlungen ist, umfassend:
- einen Mitteltrakt (2), der durch mindestens zwei gegenüberliegende Wände (10, 11) definiert ist und der darin einen Hohlraum (6) mit einer Breite (D), quer verlaufend, mindestens an einem Abschnitt aufweist; wobei die Breite (D) innerhalb des Hohlraums (6) durch den Abstand zwischen den beiden gegenüberliegenden Wänden (10, 11) definiert ist, wobei der Mitteltrakt (2) an beiden Enden mit einem ersten Anschlussende (3) und einem zweiten Anschlussende (4) endet, die gegenüberliegen;
- einen ersten Trakt (7), der an dem ersten Anschlussende (3) mittels eines Verbindungsabschnitts (21) integral mit dem Mitteltrakt (2) verbunden ist und den Mitteltrakt (2) verlängert, wobei der erste Trakt (7) mit einem Eingriffselement (8) mit einer Querbreite, zumindest für einen Trakt, der Größe (S) endet; wobei die Größe (S) vorzugsweise größer ist als die Breite (D) des Hohlraums (6);
wobei die Vorrichtung eine Ruhekonfiguration aufweist, in der sich der Mitteltrakt entlang einer Längsachse (X-X) erstreckt und das zweite Anschlussende (4) des Mitteltrakts und der erste Trakt (2, 7) voneinander entkoppelt sind, und eine aktive Konfiguration, in der der Mitteltrakt und der erste Trakt (2, 7) miteinander gekoppelt sind, wodurch der Vorrichtung eine geschlossene Ringform verliehen wird, die eine Fläche (C) innerhalb derselben schneidet, die eine kleinere Endquerschnittsfläche (B') auf dem Analkanal (CA) als die Anfangsquerschnittsfläche (B) definiert;
wobei der Mitteltrakt und der erste Trakt (2, 7) in der aktiven Konfiguration der Vorrichtung eine Durchdringungskupplung erzeugen, die durch das Einsetzen des Eingriffselements (8) in den Hohlraum (6) definiert wird,
wobei die Vorrichtung zudem Verriegelungsmittel (16) umfasst, die zwischen dem ersten Trakt und dem Mitteltrakt (7, 2) aktiv sind, um ein Gleiten dazwischen zu verhindern,
wobei das Eingriffselement (8) durch einen kugelförmigen Körper (15) mit einem diametralen Querschnitt gleich (S) definiert ist und
wobei die Verriegelungsmittel (16) auf den Mitteltrakt (2) aktiv sind, um ein Zusammendrücken des Hohlraums (6) stromaufwärts des Eingriffselements (8) zu definieren; wobei das Zusammendrücken verhindert, dass der kugelförmige Körper (15) aus dem Hohlraum (6) austritt,
und dadurch, dass der erste Trakt (7) durch einen zylindrischen Trakt (13) definiert ist, der eine Erstreckungsrichtung entlang einer Längsachse (X-X) aufweist, die mit dem Eingriffselement (8) endet, und dass sich der Hohlraum (6) über die gesamte Länge des Mitteltrakts (2) entlang einer Längsachse (X-X) desselben erstreckt, so dass die Verschiebung des kugelförmigen Körpers (8) innerhalb des Hohlraums (6) beliebig variiert werden kann und durch Einstellen der Eindringtiefe vom kugelförmigen Körper (8) innerhalb des Hohlraums (6) ist es möglich, die geschlossene ringförmige Kontur (K-K) der Vorrichtung (1) beliebig zu dimensionieren, je nachdem, wie stark man den Angangsquerschnitt (B) des Analkanals (CA) komprimieren möchte.

2. Vorrichtung nach Anspruch 1, **dadurch gekennzeichnet, dass** das Eingriffselement (8) in der aktiven Konfiguration vollständig innerhalb des Hohlraums (6) enthalten ist.

3. Vorrichtung nach Anspruch 1, **dadurch gekennzeichnet, dass** die aktive Konfiguration einen vorläufigen Schritt vorsieht, in dem der erste Trakt und der Mitteltrakt (7, 2) eine Kontaktposition erzeugen, in der das Eingriffselement (8) an mindestens einer Stelle (P) mit dem Hohlraum (6) in Kontakt kommt, wodurch eine geschlossene ringförmige Kontur (K-K) definiert wird und einen zweiten Endschritt, wobei der erste Trakt und der Mitteltrakt (7, 2) eine Zwangsposition erzeugen, in der sich das Eingriffselement (8) innerhalb des Hohlraums (6) verschiebt; die Verschiebung erfolgt entlang der Richtung (F), die zur Kontaktstelle (P) tangential ist.

4. Vorrichtung nach Anspruch 1, **dadurch gekennzeichnet, dass** der erste Trakt und der Mitteltrakt (7, 2) aus einem nachgiebigen elastischen Material, vorzugsweise Silikon, hergestellt sind.

5. Vorrichtung nach Anspruch 1, **dadurch gekennzeichnet, dass** der Mitteltrakt (2) durch einen Streifen (9) definiert ist, der eine Erstreckungsrichtung entlang einer Längsachse (X-X) aufweist, der in dessen Querschnitt eine rechteckige Kontur aufweist und eine erste Innenwand (10) und eine zweite Außenwand (11) definiert, wobei die erste und die zweite Wand (10, 11) sich innerhalb des Hohlraums (6) einen Abstand (D) ineinander schneiden.

6. Vorrichtung nach Anspruch 5, **dadurch gekennzeichnet, dass** die Innenwand (10) des Streifens (9), wenn die Vorrichtung die aktive Konfiguration einnimmt, der Innenseite der geschlossenen ringförmigen Kontur (K-K) zugewandt ist.

7. Vorrichtung nach Anspruch 1, **dadurch gekennzeichnet, dass** die Verriegelungsmittel (16) durch mindestens ein Band (17) definiert sind, das auf dem Mitteltrakt (2) aktiv ist.

8. Vorrichtung nach Anspruch 1, **dadurch gekennzeichnet, dass** die auf den Mitteltrakt (2) aktiven Verriegelungsmittel (16) eine Verkürzung des Abstands (D) fördern.

9. Vorrichtung nach Anspruch 1, **dadurch gekennzeichnet, dass** die Kopplung zwischen dem Eingriffselement (8) und dem Hohlraum (6) vom Interferenztyp ist.

## Revendications

1. Dispositif de cerclage d'un canal anal (CA), ledit canal anal (CA) ayant une extension le long d'un axe (Y-Y) et une section transversale initiale (B) et étant entouré autour de sa circonférence d'une première paroi musculaire interne (SI) et d'une seconde paroi musculaire externe (SE) entourant la précédente, comprenant :
- une voie centrale (2) définie par au moins deux parois opposées (10, 11) et ayant, à l'intérieur, une cavité (6) ayant une largeur (D) transversalement, au moins dans une partie ; ladite largeur (D) étant définie, à l'intérieur de la cavité (6), par la distance entre les deux parois opposées (10, 11), la voie centrale (2) se terminant, de chaque côté, par une première extrémité terminale (3) et par une seconde extrémité terminale (4) opposées l'une à l'autre ;
- une première voie (7), intégralement connectée à la voie centrale (2) au niveau de ladite première extrémité terminale (3) au moyen d'une partie de raccordement (21) et prolongeant la voie centrale (2), la première voie (7) se terminant par un élément d'engagement (8) ayant une largeur transversale, au moins pour une voie, de dimension (S) ; ladite dimension (S) étant de préférence plus grande que la largeur (D) de la cavité (6) ; ledit dispositif ayant une configuration de repos, dans laquelle la voie centrale évolue le long d'un axe longitudinal (X-X) et la seconde extrémité terminale (4) de la voie centrale et la première voie (2, 7) sont découplées l'une de l'autre, et une configuration active dans laquelle la voie centrale et la première voie (2, 7) sont couplées l'une à l'autre, donnant au dispositif la forme d'un anneau fermé sous-tendant à l'intérieur une zone (C), définissant sur le canal anal (CA) une zone transversale finale plus petite (B') que la zone transversale initiale (B) ;
dans lequel ladite voie centrale et ladite première voie (2, 7) forment, dans la configuration active du dispositif, un couplage interpénétrant réalisé par l'insertion de l'élément d'engagement (8) à l'intérieur de la cavité (6),
dans lequel le dispositif comprend aussi des moyens de verrouillage (16) actifs entre la première voie et la voie centrale (7, 2) pour prévenir le glissement entre elles,
dans lequel ledit élément d'engagement (8) est défini par un corps sphérique (15) ayant une section diamétrale égale à (S), et
dans lequel lesdits moyens de verrouillage (16) sont actifs sur la voie centrale (2) pour définir une compression de la cavité (6) en amont de l'élément d'engagement (8), ladite compression empêchant le corps sphérique (15) de sortir de la cavité (6),
et dans lequel ladite première voie (7) est définie par une voie cylindrique (13), ayant une direction d'extension le long d'un axe longitudinal (X-X), se terminant par l'élément d'engagement (8), et dans lequel la cavité (6) évolue le long de toute la longueur de la voie centrale (2) le long d'un axe longitudinal (X-X) de celle-ci, de sorte que la translation du corps sphérique (8) à l'intérieur de la cavité (6) peut être modifiée à souhait et, en réglant la profondeur de pénétration du corps sphérique (8) à l'intérieur de la cavité (6), il est possible de dimensionner le contour fermé en forme d'anneau (K-K) du dispositif (1) à souhait, selon la compression que l'on veut effectuer sur la section transversale initiale (B) du canal anal (CA).

2. Dispositif selon la revendication 1, **caractérisé en ce que**, dans la configuration active, ledit élément d'engagement (8) est contenu complètement à l'intérieur de la cavité (6).

3. Dispositif selon la revendication 1, **caractérisé en ce que** la dite configuration active prévoit une étape préliminaire, dans laquelle la première voie et la voie centrale (7, 2) forment une position de contact dans laquelle ledit élément d'engagement (8) entre en contact, au moins à un point (P), avec la cavité (6), définissant un contour fermé en forme d'anneau (K-K) et une seconde étape terminale, dans laquelle la première voie et la voie centrale (7, 2) forment une position de retenue, dans laquelle ledit élément d'engagement (8) se transfère à l'intérieur de la cavité (6) ; ladite translation se produisant le long de la direction (F) tangente par rapport au point de contact (P).

4. Dispositif selon la revendication 1, **caractérisé en ce que** lesdites première voie et voie centrale (7, 2) sont fabriquées en matière élastique flexible, de préférence silicone.

5. Dispositif selon la revendication 1, **caractérisé en ce que** ladite voie centrale (2) est définie par une bande (9) ayant une direction d'extension le long d'un axe longitudinal (X-X), ayant un contour rectangulaire dans sa section transversale, et définissant une première paroi interne (10) et une seconde paroi externe (11), lesdites première et seconde parois (10, 11) sous-tendant, à l'intérieur, une distance (D) à l'intérieur de la cavité (6).

6. Dispositif selon la revendication 5, **caractérisé en ce que** ladite bande (9) a, lorsque le dispositif se trouve dans la configuration active, sa paroi interne (10) tournée vers l'intérieur du contour fermé en forme d'anneau (K-K).

7. Dispositif selon la revendication 1, **caractérisé en ce que** lesdits moyens de verrouillage (16) sont définis par au moins une sangle (17) active sur la voie centrale (2).

8. Dispositif selon la revendication 1, **caractérisé en ce que** lesdits moyens de verrouillage (16), actifs sur la voie centrale (2), entraînent une réduction de la distance (D).

9. Dispositif selon la revendication 1, **caractérisé en ce que** ledit couplage entre l'élément d'engagement (8) et la cavité (6) est de type à interférence.
